# FASCICULE DE BREVET EUROPEEN

(11) **EP 3 753 712 B1**
(45) Date de publication et mention de la délivrance du brevet: **03.04.2024**
(21) Numéro de dépôt: 20180375.6
(22) Date de dépôt: 16.06.2020
(51) Int. Cl.: B29C 65/20, A61M 39/14, B29K 101/12

(54) **LAME CHAUFFANTE AVEC INDICATEUR DE SON ÉTAT D'USURE**
HEIZSCHWERT MIT ANZEIGEVORRICHTUNG SEINES VERSCHLEISSZUSTANDS
HEATING BLADE WITH INDICATOR OF ITS STATE OF WEAR

(30) Priorité: 19.06.2019 FR 1906572
(43) Date de publication de la demande: 23.12.2020
(73) Titulaire: MGA Technologies, 69380 Civrieux d'Azergues (FR)
(72) Inventeur: de MALLIARD, Hervé, 69160 Tassin la Demi-Lune (FR)
(74) Mandataire: Cabinet Laurent & Charras

(56) Documents cités:
- EP-A1- 0 812 665
- US-A- 4 647 756
- US-A- 5 525 186
- US-A- 5 871 612
- US-A- 6 132 833

## Description

La présente invention se rapporte à une lame chauffante, adaptée notamment à des opérations de thermo-soudure de tubes souples.

Elle concerne également une machine de thermo-soudure utilisant une telle lame chauffante.

Dans les domaines biomédical et pharmaceutique, il est connu de réaliser des opérations de connexion entre plusieurs tubes souples en matière thermoplastique, par exemple pour relier entre eux des contenants remplis de composés liquides, ou pour évacuer des composés liquides vers un récipient de stockage.

Ces opérations de connexion consistent à assembler par thermo-soudure deux tubes en matière thermoplastique bout à bout, en utilisant une lame chauffante.

Une telle lame chauffante comporte habituellement une partie tranchante, capable de sectionner les tubes, et un dispositif chauffant, conçu pour faire augmenter la température de la lame chauffante, afin de faire fondre au moins partiellement les extrémités des tubes sectionnés.

La connexion entre les tubes est réalisée en mettant en contact ces extrémités fondues par la lame chauffante, soudant ainsi les tubes entre eux après refroidissement de ceux-ci.

Ces opérations de connexion sont habituellement réalisées grâce à une machine de connexion intégrant notamment une lame chauffante.

Au cours de ces opérations de connexion, la lame chauffante utilisée subit une usure régulière, provoquant au fil du temps une détérioration de ses performances.

Par exemple, le contact répété entre la partie tranchante de la lame chauffante et les tubes à connecter entraîne un émoussement de celle-ci et mène donc à une diminution de la qualité et de la fiabilité de la section des tubes.

De même, les changements fréquents de température ou la projection de matière fondue issue des tubes à connecter peuvent dégrader le dispositif chauffant ou le matériau constitutif de la lame chauffante.

Ainsi, il est d'usage de définir pour chaque lame chauffante un nombre maximal d'utilisations au-delà duquel son usure ne permet plus de garantir une opération de thermo-soudure de qualité : la lame chauffante est alors obsolète et doit être remplacée.

Cependant, dans l'état de la technique, il n'existe pas de système fiable permettant de s'assurer qu'une lame chauffante obsolète ne puisse pas être utilisée pour une opération de thermo-soudure, alors même que son nombre maximal d'utilisations est dépassé.

En effet, dans le cas de lames chauffantes mono-usage, prévues pour n'être utilisées qu'une seule et unique fois, il appartient à l'opérateur réalisant les opérations de thermo-soudure grâce à une machine de connexion d'utiliser une lame chauffante neuve pour chaque soudure : en cas d'oubli ou de malveillance de sa part, la machine de connexion de dispose d'aucun système empêchant une nouvelle soudure avec une lame déjà utilisée et obsolète.

Le document US 6 132 833 décrit cependant une lame chauffante à usage unique pourvue d'un indicateur de l'usage effectif de ladite lame.

Dans le cas de lames chauffantes multi-usages, prévues pour être utilisées plusieurs fois jusqu'à une nombre maximal d'utilisations, la machine de connexion comporte habituellement un compteur permettant de comptabiliser le nombre d'utilisations d'une même lame chauffante et empêchant toute opération de thermo-soudure avec celle-ci si son nombre d'utilisations dépasse son nombre d'utilisations maximal.

Or, ce compteur est réinitialisé à chaque changement de lame chauffante dans la machine de connexion : il suffit ainsi, par exemple, de retirer une lame chauffante obsolète de la machine de connexion puis de la réinsérer immédiatement après pour réinitialiser le compteur et réaliser une opération de thermo-soudure avec cette lame chauffante usagée.

La présente invention a pour but de résoudre en tout ou partie cet inconvénient, en proposant une lame chauffante qui ne puisse pas être réutilisée dans une même machine une fois son nombre maximal d'utilisations dépassé, même en cas d'erreur ou de malveillance de son utilisateur.

Un autre but de l'invention est de proposer une lame chauffante qui ne puisse pas être réutilisée dans des machines différentes et non connectées entre elles une fois son nombre maximal d'utilisations dépassé.

Encore un autre but de l'invention est de proposer une lame chauffante qui puisse être de type mono-usage ou de type multi-usages.

Enfin, encore un autre but de l'invention est de proposer une machine de thermo-soudure adaptée pour coopérer avec une lame chauffante et empêcher toute opération de thermo-soudure lorsque celle-ci est obsolète.

A cet effet, elle propose une lame chauffante, conçue pour des opérations de thermo-soudure, présentant une partie tranchante et un dispositif de chauffe permettant d'augmenter la température de ladite lame chauffante au-delà d'une température seuil de soudure, caractérisée en ce qu'elle comporte un indicateur représentatif du nombre d'opérations de thermo-soudure réalisées par ladite lame chauffante, permettant d'attester de l'état d'usure de celle-ci.

Grâce à sa partie tranchante et son dispositif chauffant, une telle lame chauffante peut être utilisée, dans une machine de thermo-soudure adaptée, afin de réaliser des opérations de thermo-soudure, par exemple et de manière non limitative des opérations de connexion de tubes souples.

De plus, la lame chauffante selon l'invention comporte un indicateur représentatif du nombre d'opérations de thermo-soudure réalisées par celle-ci, permettant, par comparaison avec un nombre maximal d'utilisations de la lame chauffante défini lors de la conception et fabrication de celle-ci, d'évaluer son état d'usure.

Une grande variété de modes de réalisations sont possibles concernant la nature et le type de cet indicateur.

Par exemple, cet indicateur peut être visuel, l'apparence de la lame chauffante étant modifiée au fur et à mesure de son utilisation, ou bien de nature électronique ou numérique.

A chaque type d'indicateur correspond également un mode de lecture et d'interprétation de celui-ci par une machine de thermo-soudure adaptée, associée à la lame chauffante, afin de pouvoir en déduire le nombre d'opérations de thermo-soudure réalisées par la lame chauffante.

Il est important de remarquer que cet indicateur fait partie intégrante de la lame chauffante et lui est indissociable : il « accompagne » ainsi la lame chauffante tout au long de sa durée de vie, de la fabrication de cette dernière à sa destruction et au cours de toutes les opérations de soudure qu'elle effectue.

Cet indicateur « suit » également physiquement la lame chauffante au cours de ses déplacements, notamment lorsque celle-ci est utilisée successivement dans des machines de thermo-soudure différentes.

A chaque instant, il est ainsi possible de connaître objectivement l'état d'usure d'une lame chauffante donnée par la lecture de l'indicateur qui lui est en permanence associé. Ainsi, contrairement aux lames chauffantes de l'état de la technique, dont l'indicateur de leur état d'usure leur est dissocié (par exemple, dans les cas où cet indicateur consiste en un compteur faisant partie d'une machine de thermo-soudure, extrinsèque à la lame chauffante), il est beaucoup plus difficile de contourner cet indicateur pour réaliser une opération de soudure avec une lame chauffante obsolète.

En effet, lorsque la lame chauffante obsolète selon l'invention est retirée d'une machine de soudure puis réintroduite dans celle-ci, l'indicateur de son état d'usure n'est pas modifié et reste inchangé au cours de la manipulation de la lame chauffante :
la lecture de cet indicateur par la machine de thermo-soudure l'informera que cette lame chauffante est usée et ne peut être réutilisée.

De même, lorsqu'une telle lame chauffante obsolète est introduite dans une autre machine de thermo-soudure, l'indicateur de son état d'usure est transporté avec elle et reste identique : la deuxième machine de thermo-soudure empêchera également la réutilisation de cette lame chauffante obsolète.

Ainsi, le fait que l'indicateur de l'état d'usure de la lame chauffante est intrinsèque et indissociable de celle-ci permet de rendre cet indicateur plus fiable, car il représente fidèlement l'historique de l'utilisation de la lame chauffante depuis sa fabrication et permet donc d'accéder à une mesure de l'état d'usure réel de celle-ci.

Avantageusement, l'indicateur est modifié après chaque nouvelle soudure effectuée par la lame chauffante de manière automatique et irréversible, sans intervention d'un opérateur humain : cela permet de s'assurer de l'exactitude de l'indicateur, qui est alors indépendant de toute négligence ou erreur humaine et impossible à falsifier.

Selon un mode de réalisation, la lame chauffante est destinée à un usage unique et l'indicateur est constitué d'un revêtement thermochrome irréversible, dont la coloration est définitivement modifiée, passant d'une première teinte à une deuxième teinte, lorsque la température de la lame chauffante dépasse la température seuil de soudure.

Dans ce mode de réalisation, l'indicateur est de type visuel : l'état d'usure de lame est caractérisé et immédiatement identifiable (aussi bien par une machine de thermo-soudure adaptée que par un opérateur humain) par la couleur du revêtement thermochrome de la lame chauffante.

Ce revêtement thermochrome présente en effet la propriété de changer de couleur lorsqu'il est soumis à une température de référence : en choisissant un revêtement thermochrome dont la température de référence est inférieure à la température seuil de soudure (c'est-à-dire la température minimale à laquelle les opérations de soudure sont effectuées par la lame chauffante), il est certain que la teinte de ce revêtement thermochrome se trouve modifiée au cours de la première utilisation de la lame chauffante, passant d'une première teinte à une deuxième teinte.

Ainsi, ce type d'indicateur est particulièrement adapté à une lame chauffante de type mono-usage : le fait que l'indicateur présente la deuxième teinte dès la première soudure effectuée par la lame chauffante permet d'immédiatement savoir que celle-ci a été utilisée au moins une fois et est donc obsolète.

Ce type d'indicateur présente l'avantage d'être absolument infalsifiable, du fait de son irréversibilité : une fois la température de référence du revêtement thermochrome dépassée, celui-ci conserve définitivement la deuxième teinte car sa coloration de celui-ci n'est plus modifiée par sa température.

Dans un autre mode de réalisation, la lame chauffante est destinée à un usage multiple et l'indicateur comporte une mémoire adaptée pour sauvegarder le nombre d'opérations de thermo-soudure réalisées par ladite lame chauffante.

L'indicateur correspond alors directement au nombre d'opérations de thermo-soudure réalisées par la lame chauffante, sauvegardé sous forme numérique dans la mémoire. Le contenu de cette mémoire peut notamment être lu par une machine de thermo-soudure grâce à un microcontrôleur adapté.

En comparant le nombre d'opérations de thermo-soudure réalisées par une lame chauffante lu dans la mémoire de celle-ci à un nombre d'utilisations maximal de ladite lame chauffante, il est ainsi possible à la machine de thermo-soudure d'empêcher toute nouvelle utilisation de ladite lame chauffante si le nombre d'opérations de thermo-soudure réalisées par ladite lame chauffante est supérieur ou égal au nombre d'utilisations maximal de ladite lame chauffante.

Avantageusement, la mémoire est adaptée pour sauvegarder le nombre d'utilisations maximal de ladite lame chauffante.

Ce nombre d'utilisations maximal peut également être simplement lu par une machine de thermo-soudure grâce à un microcontrôleur adapté.

De la sorte, les deux grandeurs à comparer pour attester de l'état de l'usure de chaque lame chauffante selon l'invention, à savoir le nombre le nombre d'opérations de thermo-soudure réalisées par ladite lame chauffante et le nombre d'utilisations maximal de ladite lame chauffante, sont intégrées à cette lame chauffante et sont indissociables de celle-ci.

Contrairement à l'état de la technique, la machine de thermo-soudure utilisant la lame chauffante selon l'invention ne comporte donc pas de compteur permettant de comptabiliser le nombre d'utilisations d'une même lame chauffante : il suffit à cette machine de lire, avant chaque opération de thermo-soudure, dans la mémoire de la lame chauffante les valeurs du nombre d'opérations de thermo-soudure réalisées par ladite lame chauffante et du nombre d'utilisations maximal de ladite lame chauffante, puis de les comparer entre elles.

Lors de la fabrication de la lame chauffante, la valeur initiale du nombre d'opérations de thermo-soudure réalisées par ladite lame chauffante est fixée à zéro.

Il est envisagé que, après chaque utilisation de la lame chauffante dans une machine de thermo-soudure, la valeur du nombre d'opérations de thermo-soudure réalisées par ladite lame chauffante soit automatiquement incrémentée d'une unité par cette machine de thermo-soudure, assurant ainsi que cette valeur soit effectivement représentative de l'état d'usure de la lame chauffante et reflète l'historique fidèle de son utilisation.

Il est à noter qu'il est particulièrement avantageux que la mémoire de la lame chauffante soit de type « morte », afin que le contenu de celle-ci ne soit pas effacé à chaque fois que son l'alimentation électrique est interrompue entre les opérations de thermo-soudures effectuées.

Selon une possibilité, la mémoire est une mémoire morte de type EEPROM (« Electrically-Erasable Programmable Read-Only Memory »).

Il est également envisageable que la mémoire de la lame chauffante puisse sauvegarder d'autres informations utiles pour caractériser l'état d'usure de cette dernière.

Dans un mode de réalisation, la mémoire est adaptée pour sauvegarder la date de la dernière opération de thermo-soudure réalisée par ladite lame chauffante.

Selon une caractéristique, la mémoire est adaptée pour sauvegarder une durée maximale d'inutilisation de ladite lame chauffante.

Ces deux dernières grandeurs permettent de caractériser l'obsolescence d'une lame chauffante d'une deuxième manière : une lame chauffante est déclarée usée si la durée depuis sa dernière utilisation est supérieure à une durée maximale d'inutilisation de ladite lame chauffante.

Cette durée maximale d'inutilisation est une grandeur fixe, définie pour chaque lame chauffante par son fabricant lors de sa fabrication.

A chaque utilisation de la lame dans une machine de connexion, la date de date de la dernière opération de thermo-soudure réalisée par ladite lame chauffante est actualisée automatiquement par la machine de thermo-soudure, et sa nouvelle valeur correspond à la date du jour.

Pour estimer si une lame chauffante est obsolète ou non, une machine de thermo-soudure lit dans la mémoire de la lame chauffante la date de la dernière opération de thermo-soudure réalisée par ladite lame chauffante et la durée maximale d'inutilisation de ladite lame chauffante, puis effectue les étapes de comparaison suivantes :
- calcul de la durée séparant la date de la dernière opération de thermo-soudure réalisée par ladite lame chauffante et la date du jour, et
- blocage de toute opération de thermo-soudure si cette dernière durée est supérieure ou égale à la durée maximale d'inutilisation de ladite lame chauffante.

Ainsi, par la simple lecture des éléments sauvegardés dans la mémoire de la lame chauffante, il est possible de s'assurer qu'aucune lame chauffante utilisée un trop grand nombre de fois ou inutilisée depuis trop longtemps ne puisse être réutilisée dans une machine de thermo-soudure, et ce même s'il s'agit de la première utilisation de cette lame chauffante dans cette machine de thermo-soudure.

Selon une possibilité, la mémoire est adaptée pour sauvegarder un numéro de série de la lame chauffante.

Ce numéro de série, bien que ne permettant pas de caractériser l'état d'usure de la lame chauffante, permet d'identifier précisément une lame chauffante et son fabricant : il représente donc une information très importante complémentaire à l'historique d'utilisation de la lame chauffante.

Il est également envisageable que la mémoire de la lame chauffante soit adaptée pour sauvegarder de très nombreux autres éléments permettant de renseigner l'utilisateur de cette lame chauffante sur son état d'usure et son historique d'utilisation.

Par exemple, il est envisageable de sauvegarder dans la mémoire de la lame chauffante la date de chaque opération de thermo-soudure réalisée par cette lame chauffante.

Il peut également être utile de sauvegarder dans la mémoire la température atteinte par la lame chauffante lors de chaque opération de thermo-soudure.

Cette sauvegarde d'information doit cependant être réalisée dans la limite des capacités de stockage de la mémoire et peut présenter l'inconvénient de complexifier la programmation et la lecture de cette mémoire.

L'utilisation d'une mémoire disposant d'une capacité plus élevée peut également se révéler plus onéreuse.

L'invention se rapporte aussi à une machine de thermo-soudure comportant :
- une lame chauffante telle que précédemment décrite, et
- un capteur optique, adapté pour identifier la coloration du revêtement thermochrome de ladite lame chauffante,
ladite machine de thermo-soudure étant adaptée pour bloquer toute opération de thermo-soudure si la deuxième teinte est identifiée par le capteur optique.

Elle se rapporte également à une machine de thermo-soudure comportant une lame chauffante telle que précédemment décrite, ladite lame chauffante comportant une mémoire adaptée pour sauvegarder le nombre d'opérations de thermo-soudure réalisées par ladite lame chauffante ainsi qu'un nombre d'utilisations maximal de ladite lame chauffante,
ladite machine de thermo-soudure étant adaptée pour :
- comparer le nombre d'opérations de thermo-soudure réalisées par ladite lame chauffante et le nombre d'utilisations maximal de ladite lame chauffante, et
- bloquer toute opération de thermo-soudure si le nombre d'opérations de thermo-soudure réalisées par ladite lame chauffante est supérieur ou égal au nombre d'utilisations maximal de ladite lame chauffante.

Selon une possibilité, la machine de thermo-soudure comporte une interface homme-machine adaptée pour afficher les informations contenues dans la mémoire de la lame chauffante, et/ou un nombre d'opérations de thermo-soudure restantes, défini comme la différence entre le nombre d'utilisations maximal de ladite lame chauffante et le nombre d'opérations de thermo-soudure réalisées par ladite lame chauffante.

Cet affichage d'un nombre d'opérations de thermo-soudure restantes permet d'alerter préventivement l'utilisateur de la machine de thermo-soudure de l'usure de la lame chauffante, incitant celui-ci à la remplacer avant qu'elle ait atteint son nombre d'utilisations maximal.

L'invention concerna aussi une machine de thermo-soudure telle que précédemment décrite, dans laquelle la lame chauffante comporte une mémoire adaptée pour sauvegarder la date de la dernière opération de thermo-soudure réalisée par ladite lame chauffante ainsi qu'une durée maximale d'inutilisation de ladite lame chauffante, ladite machine de thermo-soudure étant adaptée pour :
- calculer la durée séparant la date de la dernière opération de thermo-soudure réalisée par ladite lame chauffante et la date du jour, et
- bloquer toute opération de thermo-soudure si ladite durée est supérieure ou égale à la durée maximale d'inutilisation de ladite lame chauffante.

D'autres caractéristiques et avantages de la présente invention apparaîtront à la lecture de la description détaillée ci-après, d'un exemple de mise en oeuvre non limitatif, faite en référence aux Figures annexées dans lesquelles :
[Fig. 1] est une vue d'une lame chauffante multi-usages selon l'invention,
[Fig. 2] est une vue éclatée d'une lame chauffante multi-usages selon l'invention,
[Fig. 3] est une vue d'une lame mono-usage selon l'invention.

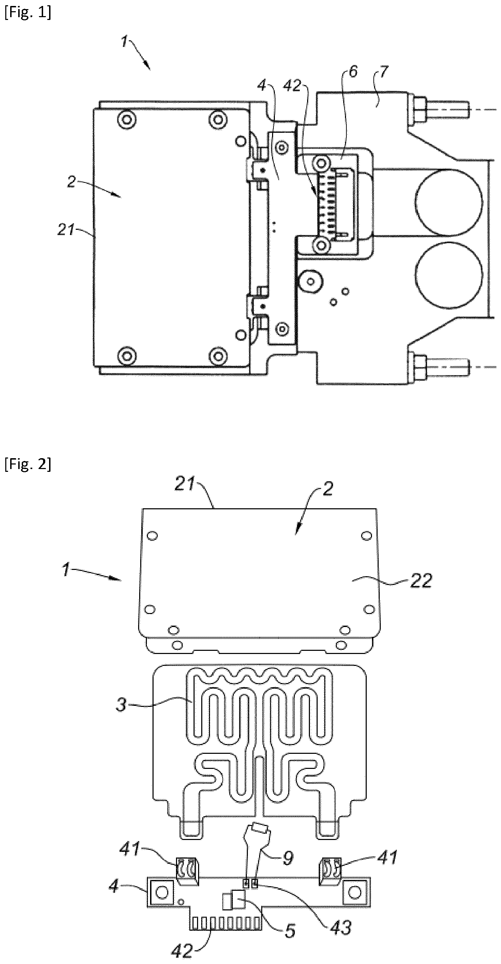

La figure 1 représente une lame chauffante 1 multi-usages selon l'invention, c'est-à-dire une lame chauffante destinée à être utilisée au cours de plusieurs opérations de thermo-soudures successives.

La lame chauffante 1 comporte une partie tranchante 2, adaptée pour sectionner par exemple des tubes souples en matière élastomère thermoplastique.

Cette partie tranchante 2 présente à cet effet un bord latéral 21 affûté, permettant de garantir une découpe rapide, nette et sans perte de matière.

Comme cela est visible sur la figure 2, représentant une vue éclatée de la lame chauffante 1, la partie tranchante 2 est formée par le pliage d'une feuille 22 sur elle-même, le bord latéral 21 correspondant ainsi à l'emplacement où est effectué ce même pliage.

La feuille 22 peut avantageusement être de nature métallique, et peut par exemple être formée d'une plaque de tôle.

La lame chauffante 1 comporte également une résistance chauffante 3, visible sur la figure 2, insérée dans la feuille 22 pliée sur elle-même : en faisant circuler dans cette résistance chauffante 3 un courant électrique, la température de celle-ci augmente par effet Joule, chauffant ainsi par conduction la partie tranchante 2 avec laquelle elle est en contact.

La résistance chauffante 3 permet donc de faire augmenter la température de la partie chauffante 2 au-delà d'une température seuil de soudure, par exemple correspondant à la température de fusion de tubes souples que la lame chauffante est destinée à sectionner, température minimale à laquelle les opérations de thermo-soudure peuvent être réalisées.

La résistance chauffante 3 est reliée à un circuit imprimé 4, solidaire de la lame chauffante 1, par l'intermédiaire de deux connecteurs 41.

Le circuit imprimé 4 comporte une mémoire 5, constituant un indicateur de l'état d'usure de la lame chauffante 1.

Cette mémoire 5 est adaptée pour sauvegarder les éléments suivants :
- le nombre d'opérations de thermo-soudure réalisées par la lame chauffante 1 depuis sa fabrication, ce nombre étant automatiquement incrémenté d'une unité à chaque utilisation de la lame chauffante 1,
- un nombre d'utilisations maximal de la lame chauffante 1,
- la date de la dernière opération de thermo-soudure réalisée par la lame chauffante 1,
- une durée maximale d'inutilisation de la lame chauffante 1, et
- un numéro de série de la lame chauffante 1, permettant de précisément identifier cette dernière et son fabricant.

La mémoire 5 est une mémoire morte de type EEPROM (« Electrically-Erasable Programmable Read-Only Memory ») : son contenu peut ainsi être lu un nombre illimité de fois par un microcontrôleur adapté et actualisé à chaque utilisation de la lame chauffante 1, sans être effacé lorsque l'alimentation électrique de la lame chauffante 1 est interrompue.

Ainsi, la mémoire 5 constitue un indicateur fidèle et fiable de l'état de l'usure de la lame chauffante 1, et est absolument indissociable de celle-ci.

En effet, comme précédemment décrit, la lecture du contenu de la mémoire 5 par une machine de thermo-soudure (non représentée), comportant un microcontrôleur adapté et à laquelle la lame chauffante 1 est connectée, permet d'empêcher toute opération de thermo-soudure lorsque la lame chauffante 1 est obsolète (c'est-à-dire lorsque l'état d'usure de celle-ci est trop important), en suivant l'un des deux processus de vérification suivants, ou une combinaison de ceux-ci.

Le premier processus utilise une définition de l'obsolescence de la lame chauffante 1 basée sur son nombre d'utilisations depuis sa fabrication : si le nombre d'opérations de thermo-soudure réalisées par la lame chauffante 1 est supérieur au nombre d'utilisations maximal de cette même lame chauffante 1, celle-ci est jugée obsolète et inutilisable.

Ces deux nombres étant à tout instant sauvegardés dans la mémoire 5, il suffit au microcontrôleur de la machine de thermo-soudure de comparer ces deux nombres puis de bloquer toute opération de thermo-soudure si le nombre d'opérations de thermo-soudure réalisées par la lame chauffante 1 est supérieur ou égal au nombre d'utilisations maximal de cette même lame chauffante 1.

Le deuxième processus utilise une définition de l'obsolescence de la lame chauffante 1 basée sur la durée d'inutilisation de la lame chauffante 1 (c'est-à-dire la durée depuis laquelle la lame chauffante 1 n'a pas été utilisée pour une opération de thermo-soudure): si cette durée d'inutilisation est supérieure à la durée maximale d'inutilisation de la lame chauffante 1, celle-ci est jugée obsolète et inutilisable.

La durée maximale d'inutilisation de la lame chauffante 1 est à tout instant sauvegardée dans la mémoire 5 et peut donc être directement lue par le microcontrôleur de la machine de thermo-soudure.

En revanche, seule la date de la dernière utilisation de la lame chauffante 1 est sauvegardée dans la mémoire 5 : la durée d'inutilisation de la lame 1 peut aisément être déduite par le microcontrôleur de la machine de thermo-soudure en comparant la date de la dernière utilisation de la lame chauffante 1 à la date du jour.

Il suffit alors au microcontrôleur de la machine de thermo-soudure de comparer cette durée d'inutilisation de la lame chauffante 1 ainsi obtenue à la durée d'inutilisation maximale, puis de bloquer toute opération de thermo-soudure si ladite durée d'inutilisation de la lame chauffante 1 est supérieure ou égale à la durée maximale d'inutilisation de cette même lame chauffante 1.

Il est également envisagé que la machine de thermo-soudure comporte une interface homme-machine adaptée pour communiquer à un utilisateur de cette dernière, par exemple par affichage sur un écran, des informations concernant l'état d'usure de la lame chauffante 1, notamment à but préventif.

Il peut ainsi être particulièrement avantageux de communiquer à cet utilisateur les éléments sauvegardés dans la mémoire 5, ainsi qu'un nombre d'opérations de thermo-soudure restantes de la lame chauffante 1, défini comme la différence entre le nombre d'utilisations maximal de ladite lame chauffante et le nombre d'opérations de thermo-soudure réalisées par ladite lame chauffante (ces deux nombres étant sauvegardés dans la mémoire 5).

Ce nombre d'opérations de thermo-soudure restantes de la lame chauffante 1 donne accès à l'utilisateur à une estimation de l'état d'sure de la lame chauffante 1, avant que celle-ci ne soit obsolète : il est donc possible à l'utilisateur d'anticiper l'obsolescence de la lame chauffante 1 et de préparer le remplacement de celle-ci.

Dans le mode de réalisation envisagé par les figures 1 à 2 précédentes, le circuit imprimé 4 de la lame chauffante 1 est adapté pour être connecté à un circuit imprimé 6, solidaire d'une machine de thermo-soudure, par l'intermédiaire des huit bornes 42 du circuit imprimé 4.

Ces bornes 42 peuvent coopérer avec un connecteur de liaison (non représenté) prévu sur le circuit imprimé 6.

Ce circuit imprimé 6 permet de connecter la lame chauffante 1 (et plus généralement toute lame chauffante adaptée pour être connectée avec le circuit imprimé 6) à une machine de thermo-soudure pour réaliser une opération de thermo-soudure.

Une fois la connexion entre la lame-chauffante 1 et le circuit imprimé 6 effectuée, il est notamment possible à un microcontrôleur de la machine de thermo-soudure de lire les éléments sauvegardés dans la mémoire 5, et de faire circuler un courant électrique dans la résistance chauffante 3 pour faire augmenter la température de la lame chauffante 1.

De plus, le circuit imprimé 6 est lui-même monté sur un élément de support 7 pouvant être déplacé par des moyens d'actionnement et de commande de la machine de thermo-soudure : par l'intermédiaire de cet élément de support 7, il est ainsi possible de déplacer la lame chauffante 1 au cours d'une opération de thermo-soudure, par exemple pour sectionner des tubes souples.

Par ailleurs, la lame chauffante 1 comporte un thermocouple 9, relié aux deux bornes 43 du circuit imprimé 4.

Ce thermocouple 9 permet de mesurer la température atteinte par la lame chauffante 1 au cours d'une opération de thermo-soudure : cette information peut être d'une grande utilité à un utilisateur de cette lame chauffante 1, lui permettant de s'assurer que la lame chauffante atteint bien la température requise, par exemple pour faciliter la section de tubes souples et pour effectuer une fusion partielle de ceux-ci.

Il est envisageable que le circuit imprimé 7 comporte un conditionneur de thermocouple numérique, permettant de transmettre la mesure du thermocouple 9 à la machine de thermo-soudure sous la forme d'un signal numérique, en utilisant un unique fil de transmission.

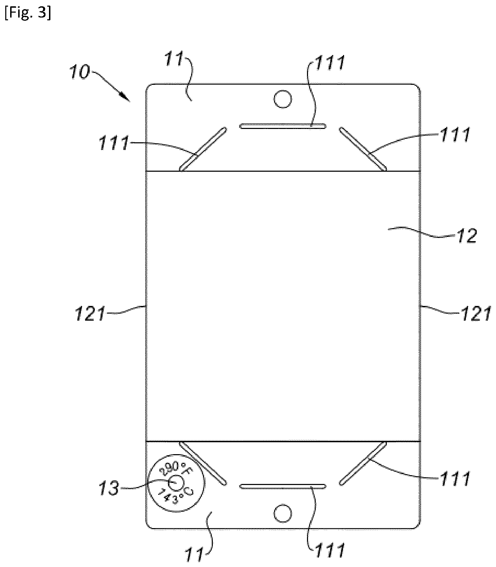

La figure 3 représente une lame chauffante 10 selon un deuxième mode de réalisation de l'invention.

Cette lame chauffante 10 est une lame chauffante mono-usage, destinée à être utilisée une seule fois au cours d'une unique opération de thermo-soudure.

Cette lame chauffante 10 présente une portion passive 11 comprenant les deux extrémités de la lame chauffante 10 dans le sens de la longueur, et une portion active 12, placée au centre de la lame chauffante 10 selon sa longueur.

La portion active 12 est formée d'un alliage chromé présentant une forte résistance électrique : en faisant circuler un courant électrique dans la lame chauffante 10, il est ainsi possible de faire augmenter la température de celle-ci par effet Joule.

Cette portion active présente deux bords latéraux 121 affûtés, adaptés pour sectionner des tubes souples en matière élastomère thermoplastiques et entraîner une fusion partielle de ceux-ci.

La portion passive 11 présente des fentes 111, permettant de connecter la lame chauffante 10 à une machine de thermo-soudure adaptée : grâce à un système de pinces (non représenté) pouvant être insérées dans les fentes 111, il est possible de déplacer la lame chauffante 10 pour effectuer des opérations de section et de fusion et d'alimenter celle-ci en courant électrique.

La portion passive 111 présente un revêtement thermochrome, se présentant sous la forme d'une pastille thermochrome 14, collée sur la portion passive 111.

Cette pastille thermochrome 14 a pour caractéristique de changer de teinte lorsque soumise à une température supérieure à une température de référence, et ce de manière irréversible : une fois la température de référence atteinte, une nouvelle baisse de la température de la pastille thermochrome 14 ne modifie plus sa teinte.

La pastille thermochrome 14 constitue ainsi un indicateur fiable de l'état de l'usure de la lame chauffante 10.

En effet, à condition que la pastille thermochrome 14 soit choisie de manière que la température de référence qui lui est associée est inférieure à la température seuil de soudure à laquelle la lame chauffante 10 est soumise lors des opérations de thermo-soudure, la coloration de la pastille thermochrome 14 permet de rapidement identifier si la lame chauffante 10 a déjà été utilisée ou non depuis sa fabrication :
- si la pastille thermochrome présente toujours sa teinte initiale, la lame chauffante 10 n'a jamais été utilisée,
- si la pastille thermochrome 14 présente une teinte différente de sa teinte initiale, la lame chauffante 10 a déjà été utilisée au moins une fois pour une opération de thermo-soudure et est considérée comme obsolète.

Il est également envisageable que, à la place de la pastille thermochrome 14, l la lame chauffante 10 présente à sa surface une couche de peinture thermochrome, présentant exactement les mêmes caractéristiques et ayant la même fonction.

Le fait que la pastille thermochrome 14 soit indissociable de la lame chauffante 10, associé au fait que son changement de teinte est automatique et irréversible, fait de cette pastille thermochrome 14 un indicateur sûr et infalsifiable de l'état d'usure de la lame chauffante 10.

Grâce à cet indicateur, il est possible de s'assurer de manière simple qu'une lame chauffante usée ne puisse pas être réutilisée dans une machine de thermo-soudure. En premier lieu, il est possible à un utilisateur de la lame chauffante 10 d'identifier la teinte de la pastille thermochrome 14, et de ne pas utiliser cette lame chauffante 10 si la pastille thermochrome 14 ne présente pas sa teinte initiale.

En deuxième lieu, il est envisagé que la machine de thermo-soudure à laquelle la lame chauffante 10 est connectée soit dotée d'un capteur optique, adapté pour identifier la coloration de la pastille thermochrome 14 : un microcontrôleur relié à ce capteur optique peut ainsi être programmé pour bloquer toute opération de thermo-soudure si la teinte de la pastille thermochrome 14 identifiée par le capteur optique ne correspond pas à la teinte initiale de celle-ci.

Ainsi, l'invention permet bien de garantir qu'une lame chauffante ne puisse pas être réutilisée dans le cadre d'une opération de thermo-soudure, aussi bien dans le cas d'une lame chauffante mono-usage que d'une lame chauffante multi-usages.

## Revendications

1. Lame chauffante (1, 10), conçue pour des opérations de thermo-soudure, présentant une partie tranchante (2) et un dispositif de chauffe (3) permettant d'augmenter la température de ladite lame chauffante (1, 10) au-delà d'une température seuil de soudure, comportant un indicateur (5, 14) non falsifiable du nombre d'opérations de thermo-soudure réalisées par ladite lame chauffante (1, 10), permettant d'attester de l'état d'usure de celle-ci, **caractérisée en ce que** la lame chauffante (1, 10) est :
• soit une lame à usage unique et dans laquelle l'indicateur (5, 14) est constitué d'un revêtement thermochrome irréversible (14), dont la coloration est définitivement modifiée, passant d'une première teinte à une deuxième teinte, lorsque la température de la lame chauffante (1, 10) dépasse la température seuil de soudure,
• soit une lame à usage multiple.

2. Lame chauffante (1, 10) selon la revendication 1, destinée à un usage multiple et dans laquelle l'indicateur (5, 14) comporte une mémoire (5) adaptée pour sauvegarder le nombre d'opérations de thermo-soudure réalisées par ladite lame chauffante (1, 10).

3. Lame chauffante (1, 10) selon la revendication précédente, dans laquelle la mémoire (5) est adaptée pour sauvegarder un nombre d'utilisations maximal de ladite lame chauffante (1, 10).

4. Lame chauffante (1, 10) selon l'une quelconque des revendications 2 et 3, dans laquelle la mémoire (5) est adaptée pour sauvegarder la date de la dernière opération de thermo-soudure réalisée par ladite lame chauffante (1, 10).

5. Lame chauffante (1, 10) selon l'une quelconque des revendications 2 à 4, dans laquelle la mémoire (5) est adaptée pour sauvegarder une durée maximale d'inutilisation de ladite lame chauffante (1, 10).

6. Lame chauffante (1, 10) selon la revendication précédente, dans laquelle la mémoire (5) est une mémoire morte de type EEPROM (« Electrically-Erasable Programmable Read-Only Memory »).

7. Machine de thermo-soudure comportant :
- une lame chauffante (1, 10) conforme à la revendication 1, et
- un capteur optique, adapté pour identifier la coloration du revêtement thermochrome (14) de ladite lame chauffante (1, 10), ladite machine de thermo-soudure étant adaptée pour bloquer toute opération de thermo-soudure si la deuxième teinte est identifiée par le capteur optique.

8. Machine de thermo-soudure comportant une lame chauffante (1, 10) conforme à l'une quelconque des revendications 2 à 6, ladite lame chauffante (1, 10) comportant une mémoire (5) adaptée pour sauvegarder le nombre d'opérations de thermo-soudure réalisées par ladite lame chauffante (1, 10) ainsi qu'un nombre d'utilisations maximal de ladite lame chauffante (1, 10),
ladite machine de thermo-soudure étant adaptée pour :
- comparer le nombre d'opérations de thermo-soudure réalisées par ladite lame chauffante (1, 10) et le nombre d'utilisations maximal de ladite lame chauffante (1, 10),
et
- bloquer toute opération de thermo-soudure si le nombre d'opérations de thermo-soudure réalisées par ladite lame chauffante (1, 10) est supérieur ou égal au nombre d'utilisations maximal de ladite lame chauffante (1, 10).

9. Machine de thermo-soudure selon la revendication précédente, comportant une interface homme-machine adaptée pour afficher les informations contenues dans la mémoire (5) de la lame chauffante (1, 10), et/ou un nombre d'opérations de thermo-soudure restantes, défini comme la différence entre le nombre d'utilisations maximal de ladite lame chauffante (1, 10) et le nombre d'opérations de thermo-soudure réalisées par ladite lame chauffante (1, 10).

10. Machine de thermo-soudure selon l'une quelconque des revendications 8 et 9, dans laquelle la lame chauffante (1, 10) comporte une mémoire (5) adaptée pour sauvegarder la date de la dernière opération de thermo-soudure réalisée par ladite lame chauffante (1, 10) ainsi qu'une durée maximale d'inutilisation de ladite lame chauffante (1, 10),
ladite machine de thermo-soudure étant adaptée pour :
- calculer la durée séparant la date de la dernière opération de thermo-soudure réalisée par ladite lame chauffante (1, 10) et la date du jour, et
- bloquer toute opération de thermo-soudure si ladite durée est supérieure ou égale à la durée maximale d'inutilisation de ladite lame chauffante (1, 10).

## Patentansprüche

1. Heizelement (1, 10), das für Thermoschweißvorgänge ausgelegt ist, wobei es einen schneidend wirkenden Teilbereich (2) sowie eine Heizvorrichtung (3) aufweist, welche es ermöglicht, die Temperatur des Heizelements (1, 10) über einen Schweißtemperaturschwellenwert hinaus zu erhöhen, wobei es eine fälschungssichere Anzeige (5, 14) der Anzahl an Thermoschweißvorgängen aufweist, welche das Heizelement (1, 10) ausgeführt hat, sodass der Abnutzungsgrad desselben festgestellt werden kann, **dadurch gekennzeichnet, dass** es sich bei dem Heizelement (1, 10) um Folgendes handelt:
• entweder um ein Element zum Einmalgebrauch, wobei die Anzeige (5, 14) in einer irreversibel thermochromen Beschichtung (14) besteht, deren Färbung sich derart endgültig verändert, dass sie ausgehend von einem ersten Farbton einen zweiten Farbton annimmt, wenn die Temperatur des Heizelements (1, 10) den Schweißtemperaturschwellenwert übersteigt,
• oder um ein Element zum Mehrfachgebrauch.

2. Heizelement (1, 10) nach Anspruch 1, das für einen Mehrfachgebrauch bestimmt ist, wobei die Anzeige (5, 14) einen Speicher (5) aufweist, der dafür geeignet ist, die Anzahl an Thermoschweißvorgängen zu speichern, welche das Heizelement (1, 10) ausgeführt hat.

3. Heizelement (1, 10) nach dem vorhergehenden Anspruch, wobei der Speicher (5) dafür geeignet ist, eine Höchstanzahl an Verwendungen des Heizelements (1, 10) zu speichern.

4. Heizelement (1, 10) nach einem beliebigen der Ansprüche 2 und 3, wobei der Speicher (5) dafür geeignet ist, das Datum des letzten Thermoschweißvorgangs zu speichern, welchen das Heizelement (1, 10) ausgeführt hat.

5. Heizelement (1, 10) nach einem beliebigen der Ansprüche 2 bis 4, wobei der Speicher (5) dafür geeignet ist, eine maximale Zeitdauer speichern, während derer das Heizelement (1, 10) unbenutzt bleiben darf.

6. Heizelement (1, 10) nach dem vorhergehenden Anspruch, wobei es sich bei dem Speicher (5) um einen Speicher der Bauart EEPROM ("Electrically-Erasable Programmable Read-Only Memory") handelt.

7. Thermoschweißgerät, aufweisend:
- ein Heizelement (1, 10) gemäß Anspruch 1, und
- einen optischen Sensor, der dafür geeignet ist, die Färbung der thermochromen Beschichtung (14) des Heizelements (1, 10) zu erkennen,
wobei das Thermoschweißgerät dafür geeignet ist, jedweden Thermoschweißvorgang zu unterbinden, wenn der optische Sensor den zweiten Farbton erkennt.

8. Thermoschweißgerät, das ein Heizelement (1, 10) gemäß einem beliebigen der Ansprüche 2 bis 6 aufweist, wobei das Heizelement (1, 10) einen Speicher (5) aufweist, der dafür geeignet, die Anzahl an Thermoschweißvorgängen, welche das Heizelement ausgeführt hat, sowie eine Höchstanzahl an Verwendungen des Heizelements (1, 10) zu speichern,
- wobei das Thermoschweißgerät dafür geeignet ist:
- die Anzahl an Thermoschweißvorgängen, welche das Heizelement (1, 10) ausgeführt hat, und die Höchstanzahl an Verwendungen des Heizelements (1, 10) zu vergleichen, und
- jedweden Thermoschweißvorgang zu unterbinden, wenn die Anzahl an Thermoschweißvorgängen, welche das Heizelement (1, 10) ausgeführt hat, gleich der Höchstanzahl an Verwendungen des Heizelements (1, 10) oder höher als diese ist.

9. Thermoschweißgerät nach dem vorhergehenden Anspruch, wobei es eine Mensch-Maschine-Schnittstelle aufweist, die dafür geeignet ist, die Informationen, welche in dem Speicher (5) des Heizelements (1, 10) enthalten sind, und/oder die Anzahl an verbleibenden Thermoschweißvorgängen anzuzeigen, wobei letztere als die Differenz zwischen der Höchstanzahl an Verwendungen des Heizelements (1, 10) und der Anzahl an Thermoschweißvorgängen, welche das Heizelement (1, 10) ausgeführt hat, definiert ist.

10. Thermoschweißgerät nach einem beliebigen der Ansprüche 8 und 9, wobei das Heizelement (1, 10) einen Speicher (5) aufweist, dafür geeignet ist, das Datum des letzten Thermoschweißvorgangs, welchen das Heizelement (1, 10) ausgeführt hat, ebenso wie eine maximale Zeitdauer zu speichern, während derer das Heizelement (1, 10) unbenutzt bleiben darf,
wobei das Thermoschweißgerät dafür geeignet ist:
- die Zeitdauer zu berechnen, welche zwischen dem Datum des letzten Thermoschweißvorgangs, welchen das Heizelement (1, 10) ausgeführt hat, und dem tagesaktuellen Datum liegt, und
- jedweden Thermoschweißvorgang zu unterbinden, wenn diese Zeitdauer gleich der maximalen Zeitdauer, während derer das Heizelement (1, 10) unbenutzt bleiben darf, oder länger als diese ist.

## Claims

1. Heating blade (1, 10), designed for heat-sealing operations, having a cutting part (2) and a heating device (3) allowing to increase the temperature of said heating blade (1, 10) beyond a threshold sealing temperature, comprising a tamper-proof indicator (5, 14) of the number of heat-sealing operations carried out by said heating blade (1, 10), allowing to attest to the state of wear thereof, **characterized in that** the heating blade (1, 10) is:
• either a single-use blade and in which the indicator (5, 14) consists of an irreversible thermochromic coating (14), the coloration of which is permanently modified, changing from a first hue to a second hue, when the temperature of the heating blade (1, 10) exceeds the sealing threshold temperature,
• or a multi-purpose blade.

2. Heating blade (1, 10) according to claim 1, intended for a multi-purpose use and wherein the indicator (5, 14) comprises a memory (5) adapted to save the number of heat-sealing operations carried out by said heating blade (1, 10).

3. Heating blade (1, 10) according to the preceding claim, wherein the memory (5) is adapted to save a maximum number of uses of said heating blade (1, 10).

4. Heating blade (1, 10) according to any one of claims 2 and 3, wherein the memory (5) is adapted to save the date of the last heat-sealing operation carried out by said heating blade (1, 10).

5. Heating blade (1, 10) according to any one of claims 2 to 4, wherein the memory (5) is adapted to save a maximum duration of non-use of said heating blade (1, 10).

6. Heating blade (1, 10) according to the preceding claim, wherein the memory (5) is an EEPROM ("Electrically-Erasable Programmable Read-Only Memory").

7. Heat sealing machine comprising:
- a heating blade (1, 10) according to claim 1, and
- an optical sensor, adapted to identify the coloration of the thermochromic coating (14) of said heating blade (1, 10),
said heat sealing machine being adapted to block any heat-sealing operation if the second hue is identified by the optical sensor.

8. Heat sealing machine comprising a heating blade (1, 10) according to any one of claims 2 to 6, said heating blade (1, 10) comprising a memory (5) adapted to save the number of heat-sealing operations carried out by said heating blade (1, 10) as well as a maximum number of uses of said heating blade (1, 10),
- said heat sealing machine being adapted to:
- compare the number of heat-sealing operations carried out by said heating blade (1, 10) and the maximum number of uses of said heating blade (1, 10), and
- block any heat-sealing operation if the number of heat-sealing operations carried out by said heating blade (1, 10) is greater than or equal to the maximum number of uses of said heating blade (1, 10).

9. Heat sealing machine according to the preceding claim, comprising a manmachine interface adapted to display the information contained in the memory (5) of the heating blade (1, 10), and/or a number of remaining heat-sealing operations, defined as the difference between the maximum number of uses of said heating blade (1, 10) and the number of heat-sealing operations carried out by said heating blade (1, 10).

10. Heat sealing machine according to any one of claims 8 and 9, wherein the heating blade (1, 10) comprises a memory (5) adapted to save the date of the last heat-sealing operation carried out by said heating blade (1, 10) as well as a maximum duration of non-use of said heating blade (1, 10),
said heat sealing machine being adapted to:
- calculate the time between the date of the last heat-sealing operation carried out by said heating blade (1, 10) and the current date, and
- block any heat-sealing operation if said duration is greater than or equal to the maximum duration of non-use of said heating blade (1, 10).
